# FASCICULE DE BREVET EUROPEEN

(11) **EP 1 605 870 B1**
(45) Date de publication et mention de la délivrance du brevet: **26.09.2012**
(21) Numéro de dépôt: 04718364.5
(22) Date de dépôt: 08.03.2004
(51) Int. Cl.: A61F 2/24

(54) **DISPOSITIF DE RENFORT INTRAPARIETAL POUR VALVE AORTIQUE ET VALVE AORTIQUE RENFORCEE**
INTRAPARIETALE AORTENKLAPPEN-VERSTÄRKUNGSVORRICHTUNG UND VERSTÄRKTE AORTENKLAPPE
INTRAPARIETAL AORTIC VALVE REINFORCEMENT DEVICE AND REINFORCED AORTIC VALVE

(30) Priorité: 21.03.2003 CH 480032003; 26.03.2003 US 457291 P
(43) Date de publication de la demande: 21.12.2005
(73) Titulaire: Leman Cardiovascular SA, 1110 Morges (CH)
(72) Inventeur: Andrieu, Raymond, 1169 Yens (CH); Kalangos, Afksendiyos, 1208 Genève (CH)
(74) Mandataire: Micheli & Cie SA
(86) Numéro de dépôt international: PCT/IB2004/000707
(87) Numéro de publication internationale: WO 2004/082537

(56) Documents cités:
- EP-A- 0 850 607
- WO-A-00/67661
- WO-A-01/30275
- US-A- 5 865 723
- US-B1- 6 461 382
- US-B1- 6 482 228

## Description

La présente invention concerne une prothèse biologique munie d'un dispositif de renfort intraparietal destiné à être intégré dans cette prothèse biologique.

La chirurgie cardiaque connaît une évolution constante due à l'avancée technique au niveau des matériels et des techniques utilisés. Notamment, les prothèses valvulaires pour le coeur font l'objet de recherches et plusieurs types de ces prothèses sont actuellement disponibles.

On connaît d'abord les prothèses mécaniques consistant essentiellement en une pièce métallique comprenant une cage et les clapets constituant la valve proprement dite ainsi qu'en une couronne de tissu synthétique comme le Téflon permettant de solidariser le dispositif avec le pourtour de l'orifice à remplacer. Malgré sa durée de vie importante, ce type de prothèses a des désavantages non négligeables, en particulier la nécessité d'un traitement anticoagulant du patient pendant toute sa vie.

Il existe également des prothèses biologiques qui permettent, entre autre, de supprimer ce traitement anticoagulant. Elles sont souvent des valves animales prélevées essentiellement sur le porc et traitées ensuite par un procédé adapté afin de les préparer pour l'implantation dans le corps humain. Actuellement, on peut regrouper ces prothèses biologiques en deux différentes catégories, les prothèses stentées d'une part et les prothèses non-stentées d'autre part.

Les premières consistent en une valvule biologique, par exemple la valvule aortique porcine ou une valve biologique reconstruite à partir du péricarde bovin, et en une couronne rigide. Cette couronne est réalisée en un matériau adapté comme le Titanium couvert d'un tissu comme le Téflon et entoure la valvule à laquelle elle est fixée. Ainsi, elle sert, d'une part, comme support de la valvule biologique de manière à la maintenir sur place et dans sa forme une fois implantée et, d'autre part, comme point d'encrage sur lequel sont placés les points de suture qui fixent la prothèse biologique stentée à l'orifice à équiper avec la valvule. La couronne est donc un élément intermédiaire artificiel entre les parois naturelles de l'orifice et la valvule biologique implantée. De sa conception, elle permet donc de réaliser l'implantation de la prothèse biologique préparée préalablement de façon simplifiée dans le sens qu'elle ne nécessite qu'un seul plan de suture autour de son pourtour au niveau de l'anneau de la valve native à remplacer, la prothèse biologique étant fixée et maintenue en place par ce support, le stent. Par contre, hormis de permettre cette technique d'implantation relativement simple et avantageuse, la présence de cette couronne entraîne également des désavantages importants, notamment du fait que la prothèse biologique comprenant cette couronne rigide comme stent est placée dans l'orifice naturel, l'espace disponible pour la valvule de remplacement est diminué par rapport à la valve humaine originale par la surface de la circonférence occupée par le stent. Par conséquent, le gradient de pression dans la valve de remplacement est artificiellement augmenté par la présence du stent.

De ce fait, on utilise maintenant également des prothèses biologiques non-stentées comportant seulement un tissu synthétique comme le Téflon cousu autour de son pourtour lors du procédé de préparation d'une telle prothèse ou un tissu biologique comme le péricarde préalablement traité. Ces deux types de tissus occupent sensiblement moins de volume qu'un stent du type décrit ci-haut. Le gradient de pression dans la valve de remplacement ressemble ainsi plus à la valeur naturelle. Du fait de la souplesse augmentée de ce type de prothèse biologique par rapport aux prothèses biologiques stentées ainsi que par rapport aux prothèses mécaniques, il présente également un avantage au niveau de l'hémodynamique à travers l'orifice équipé de la valvule de remplacement. Par contre, ces avantages sont contrebalancés par le fait que la complexité de la technique et donc le temps d'implantation d'une telle prothèse non stentée sont considérablement augmentés. En effet, l'absence d'un stent maintenant la valvule biologique en place et lui rendant la stabilité nécessaire oblige le chirurgien de placer, lors de l'implantation, une suture supplémentaire autour du pourtour de la valvule en position sous coronarienne. En plus, la prothèse biologique doit être implantée, par exemple dans le cas d'un remplacement de la valvule aortique, dans une orientation précise mais peu visible pour le chirurgien du fait qu'elle est placée à l'intérieur de l'orifice naturel. Si la structure rigide du stent peut servir dans le cas d'une prothèse biologique stentée comme moyen de repérage de l'orientation nécessaire, l'implantation est rendue d'autant plus difficile dans le cas des prothèses biologiques non-stentées du fait de l'absence de ce repère.

Le document WO 0067661 décrit plusieurs appareils comprenant une valve cardiaque de remplacement et un dispositif de positionnement et de maintien de la valve de remplacement comportant un certain nombre d'éléments amovibles permettant de stabiliser les commissures de la valve de remplacement. Dans certaines formes d'exécution présentées dans ce document, un dispositif de positionnement et de maintien de la valve de remplacement comprend un anneau sur lequel sont fixés au moyen de sutures ou d'agrafes un certain nombre d'éléments stabilisateurs. Chacun de ces éléments stabilisateurs est formé d'une partie allongée, possiblement courbée pour suivre la courbure de la paroi interne de l'aorte du patient. Tous les éléments stabilisateurs formant le dispositif sont interconnectés via l'anneau.

Les éléments stabilisateurs et l'anneau peuvent être fixés de plusieurs manière à la valve cardiaque de remplacement : cousus ou agrafés directement sur le tissu de la valve cardiaque de remplacement, glissés dans des poches, elles-mêmes cousues sur le tissu de la valve cardiaque de remplacement, cousus ou agrafés sur un tissu couvrant synthétique lui-même cousu sur le tissu de la valve cardiaque de remplacement.

Les dispositifs décrits dans le document WO 0067661 permettent de rigidifier la valve de remplacement et ainsi une mise en place facilitée pour le chirurgien. Cependant, ils comportent tous une strucuture extérieure à la valve de remplacement, cousue ou agrafée sur le tissu biologique, la présence d'une telle structure extérieure entraînant une diminution de l'espace disponible pour le flux sanguin. Par conséquent, le gradient de pression dans la valve de remplacement est artificiellement augmenté par la présence d'un dispositif tel que décrit dans WO 0067661.

Le but de la présente invention est d'obvier aux inconvénients précités des moyens actuels et de créer un dispositif de renfort pour des prothèses biologiques permettant la réalisation des prothèses biologiques réunissant les avantages à la fois des prothèses biologiques conventionnelles stentées et non-stentées, notamment de réaliser des prothèses biologiques étant rigidifiées et maintenues dans leur forme voulue sans recours à un stent traditionnel et disposant d'un maximum de la surface voire du volume disponible pour leur fonction primaire, ceci tout en pouvant appliquer la technique d'implantation relativement simple et rapide des prothèses biologiques stentées actuelles à ce nouveau type de prothèses biologiques renforcées.

Ainsi, la présente invention a pour objet un dispositif de renfort intraparietal pour prothèses biologiques comprenant les caractéristiques énoncées à la revendication 1, le dispositif étant notamment adapté à être placé à l'intérieur du tissu organique de cette prothèse biologique et à renforcer la structure de cette dernière de manière à maintenir sa forme après implantation, ainsi que des prothèses biologiques munies d'au moins un dispositif de ce genre, comme énoncé à la revendication 11 et suivantes.

Le dispositif comprend en particulier une tige intraparietale adaptée pour être insérée dans le tissu organique de la prothèse biologique et une jambe fixée à une première extrémité de la tige.

Par ces mesures, on obtient un dispositif adapté à rendre une prothèse biologique, sans recours à un stent conventionnel volumineux, suffisamment stable et rigide pour la maintenir dans sa forme souhaitée, le gradient de pression dans la valvule de remplacement étant, du fait de la surface disponible accrue, plus comparable à l'état naturel préalable et de ce fait prolongeant la durée de vie de la prothèse biologique, comme pour une prothèse biologique non stentée, tout en ouvrant la possibilité de transposer la technique d'implantation favorable des prothèses biologiques stentées au nouveau type de prothèses biologiques ainsi créés.

D'autres avantages ressortent des caractéristiques exprimées dans les revendications dépendantes et de la description exposant ci-après l'invention plus en détail à l'aide de dessins.

Les dessins annexés illustrent schématiquement et à titre d'exemple, plusieurs formes d'exécution de l'invention.
Les figures 1a-c illustrent schématiquement le principe et trois formes d'exécution différentes d'un dispositif de renfort intraparietal pour prothèses biologiques.
La figure 2 est une vue schématique d'une prothèse biologique munie des dispositifs de renfort intraparietal et placée, à titre d'exemple, entre la racine aortique et l'aorte ascendante.

L'invention va maintenant être décrite en détails en référence aux dessins annexés. La description va se référer, à titre d'exemple et afin de simplifier les explications, mais sans limiter l'application de la présente invention à ce cas de figure, notamment à une prothèse biologique renforcée à l'aide des dispositifs selon l'invention et particulièrement adaptée pour le remplacement de la valve aortique du coeur.

Cette même valve peut être également implantée en position mitrale ou tricuspide à condition que l'on respecte la direction du flux sanguin.

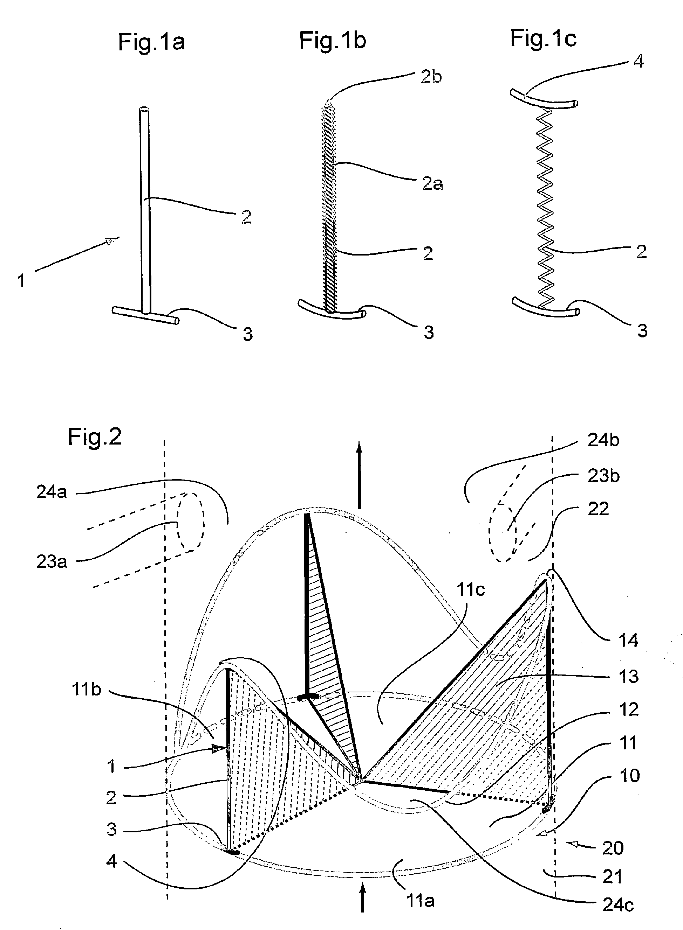

La figure 1 a montre une première forme d'exécution d'un dispositif de renfort intraparietal 1 destiné à être intégré dans une prothèse biologique selon la présente invention. Ce dispositif comprend une partie centrale réalisée par une tige intraparietale 2 qui est adaptée pour être insérée dans le tissu organique de la prothèse biologique et une première partie terminale réalisée par une jambe 3 fixée perpendiculairement à la partie centrale à une première extrémité de la tige 2. Le dispositif, tout au moins sa partie centrale, est donc adapté à être placé à l'intérieur du tissu organique d'une prothèse biologique et permet ainsi de renforcer la structure de cette dernière de manière à maintenir sa forme après implantation.

La partie centrale du dispositif 1 peut être, comme le montre la figure 1 a, formée par une tige 2 toute droite et simple. Elle peut également prendre la forme d'une tige intraparietale 2 ayant sur sa surface une partie hélicoïdale 2a en forme d'un pas de vis, comme représenté sur la figure 1 b. Cette partie centrale pourrait aussi être formée par une tige 2 avec une forme hélicoïdale, de manière à former un « tire-bouchon en miniature », comme cela est illustré schématiquement à la figure 1 c. Ces deux dernières variantes sont intéressantes car elles permettent de stabiliser le dispositif 1 dans la position dans laquelle il a été introduit dans le tissu de la prothèse biologique.

Sur la deuxième extrémité opposée à la première extrémité portant la jambe 3 susmentionnée, la tige 2 peut comprendre une partie pointue 2b adaptée pour percer et pour pénétrer, sans causer de dommages, le tissu organique de la prothèse biologique. Ce cas de figure est, à titre d'exemple, illustré à la figure 1b, mais les formes d'exécution des figures 1 a et 1 c peuvent également comporter cette caractéristique facilitant l'insertion du dispositif de renfort intraparietal 1 dans le tissu organique de la prothèse biologique.

Comme le montre la figure 1c, le dispositif peut, en plus, comprendre une deuxième partie terminale sous forme d'une attache 4 adaptée pour être fixée sur la deuxième extrémité de sa partie centrale. Cette attache joue, notamment dans le cas où la deuxième extrémité comprend une partie pointue 2b, le rôle d'un capuchon afin de recouvrir cette partie pointue 2b et de garantir la stabilité du dispositif 1 dans la position dans laquelle il a été introduit dans le tissu organique de la prothèse biologique. L'attache 4 peut être tout droite ou peut être réalisée par une barre courbée, la courbure correspondant à la courbure de la circonférence extérieure de la prothèse biologique au niveau du plan d'intersection où l'attache doit être placée.

La même remarque s'applique à la jambe 3 fixée sur la première extrémité de la partie centrale du dispositif, qui peut être réalisée simplement par une barre droite ou par une barre courbée, la courbure correspondant à la courbure de la circonférence extérieure de la prothèse biologique au niveau du plan d'intersection où la jambe 3 doit être placée, qui correspond normalement, dans le cas susmentionné à une prothèse biologique pour une valve aortique, ou mitrale, tricuspide, environ au plan dans lequel se trouve cette valve.

Le dispositif de renfort intraparietal 1 est réalisé en un matériau adapté pour garantir une stabilité suffisante tout en ayant une certaine souplesse, comme un polymère souple ou/et semi-rigide et/ou rigide, ou un métal souple comme le Titanium.

Après avoir décrit le dispositif 1 en tant que tel, va suivre maintenant, en référence à la figure 2, une description détaillée d'un exemple d'une prothèse biologique renforcée à l'aide de ce genre de dispositif. Cette figure montre une vue schématique d'une prothèse biologique pour le remplacement de la valve aortique 10 munie des dispositifs de renfort intraparietal 1 selon la présente invention et placée dans l'aorte 20, entre la racine aortique 21 et l'aorte ascendante 22.

Comme représenté à la figure 2, la prothèse biologique pour le remplacement de la valve aortique 10 susmentionnée est munie d'au moins un dispositif de renfort intraparietal 1 d'une forme d'exécution décrite ci-dessus, et dans le cas spécifique de préférence de trois dispositifs 1. En ce qui concerne la partie biologique de la prothèse 10, il s'agit dans la plupart des cas d'une valvule aortique porcine qui est équipée, lors d'un traitement préalable avant l'implantation, de dispositifs de renfort intraparietal 1.

L'agencement du dispositif de renfort intraparietal 1 et son emplacement dans la prothèse biologique renforcée se comprend facilement en vue de la structure naturelle de la valvule aortique porcine à équiper correspondant à la valvule humaine à remplacer. Cette valvule aortique voire la prothèse biologique, représentée schématiquement à la figure 2, comprend essentiellement trois feuillets 11 a, 11 b et 11 c formant le plan de la valvule, une paroi externe tubulaire 12 faisant, originalement, partie de la paroi tubulaire de l'aorte animale et entourant le plan formé par les feuillets, ces dernières étant à leur extrémité extérieure solidaires de cette paroi externe tubulaire 12 , ainsi que pour chacun des feuillets 11 a, 11 b et 11 c des parois verticales sensiblement triangulaires appelées commissures 13 qui s'étendent vers le centre de la valve ou de la cavité cardiaque pour les positions mitrale ou tricuspide, et qui sont solidaires, d'une part, des deux extrémités des feuillets 11 a, 11 b et 11 c orientées à l'intérieur de la valve ou de la cavité cardiaque pour les positions mitrale ou tricuspide et, d'autre part, de la paroi externe tubulaire 12. Ainsi, le flux du sang peut se propager dans le sens de la racine aortique 21 vers l'aorte ascendante 22, ou de l'oreillette vers la cavité ventriculaire correspondante, comme indiqué par les flèches dans la figure 2, tandis que ceci n'est pas possible dans le sens inverse. La valvule aortique porcine servant comme partie biologique de la prothèse biologique renforcée 10 est prélevée de l'aorte animale et, dans le cas d'un remplacement de la valve aortique décrit ici à titre d'exemple, la paroi externe tubulaire 12 est normalement coupée dans une forme sinusoïdale afin, d'une part, d'intégrer les jonctions sino-tubulaires 14 correspondant aux points de contact les plus élevés par rapport au plan de la valvule, entre le paroi externe tubulaire 12 et les commissures 13 et, d'autre part, de ménager de l'espace pour l'artère coronaire droite 23a respectivement l'artère coronaire gauche 23b débouchant au niveau du sinus de Valsalva droit 24a respectivement du sinus de Valsalva gauche 24b. La partie de cette paroi 12 en face du sinus de Valsalva non-coronaire 24c peut également comporter une forme sinusoïdale.

Etant donné cette configuration naturelle de la valvule aortique, la tige intraparietale 2 des dispositifs de renfort intraparietal 1 est placée à l'intérieur de la paroi externe tubulaire 12 de la valvule le long des lignes d'intersection de cette paroi avec les commissures 13 de la valvule. Cette disposition est illustrée dans la figure 2, la jambe 3 du dispositif 1 étant placée dans la partie inférieure de la prothèse au niveau du plan de la valvule, point qui sert également comme point d'insertion du dispositif dans le tissu de la prothèse biologique, et l'attache 4 étant, le cas échéant, placée environ au niveau de la jonction sino-tubulaire 14. La longueur de la partie centrale du dispositif 1 dépend de la taille de la valve aortique, mitrale ou tricuspide à équiper et est normalement compris entre 3 et 30 mm. Elle peut être choisie de façon à correspondre à la longueur de la ligne d'intersection des commissures 13 avec la paroi externe tubulaire 12 de la valvule, peut être légèrement plus longue afin de dépasser de quelques millimètres la jonction sino-tubulaire 14 ou peut également être légèrement plus courte. La jambe 3 et l'attache 4 s'étendent latéralement et perpendiculairement de la tige 2 d'environ 1 à 4 mm afin de longer la circonférence périphérique de la prothèse biologique 10. L'épaisseur des éléments du dispositif est de quelques dixièmes d'un millimètre. Les dispositifs de renfort intraparietal 1 permettent ainsi de rendre la valvule aortique porcine et surtout les commissures 13 et la paroi externe tubulaire 12 suffisamment stables pour maintenir leur forme après l'implantation.

La jambe 3 et/ou, si présente, l'attache 4 des dispositifs de renfort intraparietal 1 sont/est couvertes par un tissu en Téflon, comme peut l'être tout le pourtour extérieur de la paroi externe tubulaire 12 de la prothèse biologique renforcée 10. La première mesure permet, entre autre, de garantir la séparation des éléments du dispositif de renfort intraparietal 1 du circuit sanguin, la deuxième mesure permet de disposer d'une grande surface d'encrage pour la suture appliquée lors de l'implantation de la prothèse biologique renforcée 10 dans le corps humain.

Un dispositif de renfort intraparietal selon la présente invention peut être utilisé pour toute prothèse biologique nécessitant son renfort par des moyens occupant le moins d'espace possible afin de disposer de cet espace pour sa fonction primaire, comme la fonction valvulaire dans l'exemple décrit ci-dessus, et son application n'est donc pas limitée à cet exemple d'une prothèse biologique pour le remplacement de la valvule sigmoïde de l'aorte, mitrale ou tricuspide.

Ainsi, une prothèse biologique 10 renforcée à l'aide des dispositifs de renfort intraparietal 1 permet, de par sa conception, de réaliser l'implantation de la prothèse biologique 10, préparée et équipée préalablement, de façon relativement simple et rapide dans le sens qu'elle ne nécessite qu'une seule suture autour de son pourtour au niveau du plan de l'anneau valvulaire, celle-ci et surtout les commissures 13 étant rigidifiées et maintenues en place par les dispositifs 1, de manière analogue au cas des prothèses biologiques stentées. Comme la prothèse biologique doit être implantée, surtout dans le cas d'un remplacement de la valvule aortique, dans une orientation précise mais peu visible pour le chirurgien du fait qu'elle est placée à l'intérieur de l'orifice naturel, les dispositifs de renfort intraparietal 1 peuvent également servir comme moyen de repérage de l'orientation nécessaire, facilitant ainsi l'implantation. De plus, l'espace disponible pour la valvule de remplacement est, dû à l'absence d'une structure rigide volumineuse comme le stent conventionnel, similaire à celui de la valve humaine originale. Par conséquent, le gradient de pression dans la valve de remplacement est également similaire à sa valeur naturelle, au lieu d'être artificiellement augmenté par la présence d'un stent. En plus, du fait de la souplesse d'une telle prothèse biologique renforcée par rapport aux prothèses biologiques stentées ainsi que par rapport aux prothèses artificielles, elle présente un avantage au niveau de l'hémodynamique à travers l'orifice équipé de cette valvule de remplacement.

La présente invention permet donc de créer un dispositif de renfort intraparietal pour des prothèses biologiques permettant la réalisation des prothèses biologiques réunissant les avantages à la fois des prothèses biologiques conventionnelles stentées et non-stentées, notamment de réaliser des prothèses biologiques étant rigidifiées et maintenues dans leur forme voulue sans recours à un stent traditionnel et disposant d'un maximum de la surface voire du volume disponible pour leur fonction primaire, ceci tout en pouvant transposer la technique d'implantation relativement simple et rapide des prothèses biologiques stentées actuelles à ce nouveau type de prothèses biologiques renforcées.

## Revendications

1. Prothèse biologique (10) comprenant une valve cardiaque animale présentant une paroi tubulaire externe (12) à travers laquelle passe un flux sanguin unidirectionnel, trois feuillets (11a, 11b, 11c) solidaires de la paroi externe tubulaire à leur extrémité extérieure et pour chacun des feuillets des commissures verticales et sensiblement triangulaires **caractérisée par le fait qu'**elle comporte au moins un dispositif de renfort intrapariétal (1) formé d'une tige intrapariétale (2) implantée dans cette paroi tubulaire externe de cette valve cardiaque animale, cette tige pénétrant à l'intérieur du tissu organique de cette paroi externe de la valve cardiaque animale et s'étendant sensiblement parallèlement à ce flux sanguin ; et **par le fait que** le dispositif de renfort intrapariétal comprend une jambe (3) adaptée à être fixée à une première extrémité de la tige intrapariétale (2).

2. Prothèse biologique (10) selon la revendication 1 **caractérisée par le fait que** le dispositif de renfort intrapariétal comprend en plus une attache adaptée à être fixée sur une deuxième extrémité de la tige intrapariétale (2).

3. Prothèse biologique (10) selon l'une des revendications précédentes, **caractérisée par le fait que** la tige intraparietale (2) du dispositif de renfort intrapariétal (1) est droite.

4. Prothèse biologique (10) selon l'une des revendications précédentes, **caractérisée par le fait que** la tige intraparietale (2) du dispositif de renfort intrapariétal (1) a une forme hélicoïdale.

5. Prothèse biologique (10) selon l'une des revendications précédentes, **caractérisée par le fait que** la tige intrapariétal (2) du dispositif de renfort intrapariétal (1) comprend sur sa surface une partie hélicoïdale (2a).

6. Prothèse biologique (10) selon l'une des revendications précédentes, **caractérisée par le fait que** la tige (2) du dispositif de renfort intrapariétal (1) comprend à sa deuxième extrémité une partie pointue (2b) adaptée à percer et à pénétrer, sans causer des dommages, le tissu organique de la paroi externe tubulaire de la valve cardiaque animale.

7. Prothèse biologique (10) selon l'une des revendications précédentes, **caractérisée par le fait que** la jambe (3) adaptée à être fixée sur une première extrémité de la tige intrapariétale (2) du dispositif de renfort intrapariétal est réalisée par une barre droite.

8. Prothèse biologique (10) selon l'une des revendications précédentes, **caractérisée par le fait que** la jambe (3) adaptée à être fixée sur une première extrémité de la tige intrapariétale (2) du dispositif de renfort intrapariétal est réalisée par une barre courbée, la courbure correspondant à la courbure de la circonférence extérieure de la valve cardiaque animale.

9. Prothèse biologique (10) selon l'une des revendications 2 à 8, **caractérisée par le fait que** l'attache (4) adaptée à être fixée sur une deuxième extrémité de la tige intrapariétale (2) du dispositif de renfort intrapariétal est réalisée par une barre courbée, la courbure correspondant à la courbure de la circonférence extérieure de la valve cardiaque animale.

10. Prothèse biologique (10) selon la revendication 1, **caractérisée par le fait que** la tige intraparietale (2) du dispositif de renfort intraparietal (1) est placée à l'intérieur de la paroi externe tubulaire (12) de la valve cardiaque animale le long des lignes d'intersection de cette paroi (12) avec les commissures (13) de la valve cardiaque animale.

11. Prothèse biologique (10) selon l'une des revendications 1,2, 7, 8 et 9, **caractérisée par le fait que** la jambe (3) et/ou l'attache (4) adaptée à être fixée aux extrémités de la tige intrapariétale (2) du dispositif de renfort intrapariétal sont/est couvertes par un tissu en téflon.

12. Prothèse biologique (10) selon la revendication 11 **caractérisée par le fait que** le dispositif de renfort intrapariétal, notamment la tige intrapariétale (2), est entièrement enfoui dans le tissu biologique de la valve cardiaque animale et n'est jamais en contact avec le flux sanguin unidirectionnel passant à travers la paroi externe tubulaire de la valve cardiaque animale.

13. Prothèse biologique (10) selon la revendication 12, **caractérisée par le fait que** la totalité du dispositif de renfort intrapariétal est entièrement enfoui dans le tissu biologique de la valve cardiaque animale et n'est jamais en contact avec le flux sanguin unidirectionnel passant à travers la paroi externe tubulaire de la valve cardiaque animale

## Claims

1. Biological prosthesis (10) comprising an animal cardiac valve having a tubular outside wall (12) through which flows a unidirectional blood flow, three leaflets (11a, 11b, 11c) integral with the tubular outside wall at their outside end and for each of the leaflets vertical and essentially triangular commissures, **characterized in that** it comprises at least one intraparietal reinforcement device (1) formed by an intraparietal rod (2) implanted in this tubular outside wall of the animal cardiac valve, the said rod penetrating inside the organic tissue of this tubular outside wall of the animal cardiac valve and extending essentially parallel to said blood flow; and by the fact the intraparietal reinforcement device comprises a leg (3) designed to be attached at a first end of the intraparietal rod (2).

2. Biological prosthesis (10) according to claim 1, **characterized in that** the intraparietal reinforcement device further comprises an attachment that is suitable for being attached to a second end of the intraparietal rod (2).

3. Biological prosthesis (10) according to any one of the preceding claims, **characterized in that** the intraparietal rod (2) of the intraparietal reinforcement device (1) is straight.

4. Biological prosthesis (10) according to any one of the preceding claims, **characterized in that** the intraparietal rod (2) of the intraparietal reinforcement device (1) has a helical shape.

5. Biological prosthesis (10) according to any one of the preceding claims, **characterized in that** the intraparietal rod (2) of the intraparietal reinforcement device (1) comprises a helical portion (2a) on its surface.

6. Biological prosthesis (10) according to any one of the preceding claims, **characterized in that** the rod (2) of the intraparietal reinforcement device (1) comprises at its second end a pointed portion (2b) that is adapted for piercing and penetrating, without causing damage, the organic tissue of tubular outside wall of the animal cardiac valve.

7. Biological prosthesis (10) according to any one of the preceding claims, **characterized in that** the leg (3) designed to be attached at a first end of the intraparietal rod (2) of the intraparietal reinforcement device is made by a straight bar.

8. Biological prosthesis (10) according to any one of the preceding claims, **characterized in that** the leg (3) designed to be attached at a first end of the intraparietal rod (2) of the intraparietal reinforcement device is made by a curved bar, whereby the curvature corresponds to the curvature of the outside circumference of the animal cardiac valve.

9. Biological prosthesis (10) according to any one of claims 2 to 8, **characterized in that** the attachment (4) that is suitable for being attached to a second end of intraparietal rod (2) of the intraparietal reinforcement device is made by a curved bar, whereby the curvature corresponds to the curvature of the outside circumference of the animal cardiac valve.

10. Biological prosthesis (10) according to claim 1, **characterized in that** the intraparietal rod (2) of the intraparietal reinforcement devices (1) is placed inside tubular outside wall (12) of the animal cardiac valve along the lines of intersection of this wall (12) with the commissures (13) of the animal cardiac valve.

11. Biological prosthesis (10) according to any one of claims 1, 2, 7, 8 and 9, **characterized in that** the leg (3) and/or the attachment (4) suitable for being attached to the ends of the intraparietal rod (2) of the intraparietal reinforcement devices are/is covered by a Teflon material.

12. biological prosthesis (10) according to claim 11, **characterized in that** the intraparietal reinforcement device, especially the intraparietal rod (2), is integrally buried inside the biological tissue of the animal cardiac valve and is never in contact with the unidirectional blood flow passing through the tubular outside wall of the animal cardiac valve.

13. Biological prosthesis (10) according to claim 12, **characterized in that** the entirety of the intraparietal reinforcement device is integrally buried inside the biological tissue of the animal cardiac valve and is never in contact with the unidirectional blood flow passing through the tubular outside wall of the animal cardiac valve.

## Patentansprüche

1. Biologische Prothese (10), umfassend eine tierische Herzklappe, die eine röhrenförmige äußere Wand (12) aufweist, durch welche ein einseitig gerichteter Blutstrom fließt, drei Platten (11a, 11b, 11c), die an ihrem äußeren Ende mit der röhrenförmigen äußeren Wand verbunden sind und für jede Platte vertikale und im Wesentlichen dreieckige Verbindungen, **dadurch gekennzeichnet, dass** sie mindestens eine intraparietale Verstärkungsvorrichtung (1) umfasst, die aus einem intraparietalen Stiel (2) gebildet ist, der in die äußere röhrenförmige Wand der tierischen Herzklappe eingesetzt ist, wobei der Stiel in das Innere des organischen Gewebes der äußeren Wand der tierischen Herzklappe eindringt und sich im Wesentlichen parallel zu dem Blutstrom erstreckt; sowie dadurch, dass die intraparietale Verstärkungsvorrichtung einen Fuß (3) umfasst, der geeignet ist, an einem ersten Ende des intraparietalen Stiels (2) befestigt zu werden.

2. Biologische Prothese (10) nach Anspruch 1, **dadurch gekennzeichnet, dass** die intraparietale Verstärkungsvorrichtung ferner ein Verbindungsstück umfasst, das geeignet ist, an einem zweiten Ende des intraparietalen Stiels (2) befestigt zu werden.

3. Biologische Prothese (10) nach einem der vorhergehenden Ansprüche, **dadurch gekennzeichnet, dass** der intraparietale Stiel (2) der intraparietalen Verstärkungsvorrichtung (1) gerade ist.

4. Biologische Prothese (10) nach einem der vorhergehenden Ansprüche, **dadurch gekennzeichnet, dass** der intraparietale Stiel (2) der intraparietalen Verstärkungsvorrichtung (1) eine schraubenartige Form aufweist.

5. Biologische Prothese (10) nach einem der vorhergehenden Ansprüche, **dadurch gekennzeichnet, dass** der intraparietale Stiel (2) der intraparietalen Verstärkungsvorrichtung (1) auf seiner Oberfläche einen schraubenartigen Teil (2a) aufweist.

6. Biologische Prothese (10) nach einem der vorhergehenden Ansprüche, **dadurch gekennzeichnet, dass** der Stiel (2) der intraparietalen Verstärkungsvorrichtung (1) an seinem zweiten Ende einen spitzen Teil (2b) umfasst, der geeignet ist, das organische Gewebe der röhrenförmigen äußeren Wand der tierischen Herzklappe zu durchbohren und zu durchdringen, ohne Schäden zu verursachen.

7. Biologische Prothese (10) nach einem der vorhergehenden Ansprüche, **dadurch gekennzeichnet, dass** der Fuß (3), der geeignet ist, an einem ersten Ende des intraparietalen Stiels (2) der intraparietalen Verstärkungsvorrichtung befestigt zu werden, durch einen geraden Stab ausgeführt ist.

8. Biologische Prothese (10) nach einem der vorhergehenden Ansprüche, **dadurch gekennzeichnet, dass** der Fluß (3), der geeignet ist, an einem ersten Ende des intraparietalen Stiels (2) der intraparietalen Verstärkungsvorrichtung befestigt zu werden, durch einen gekrümmten Stab ausgeführt ist, wobei die Krümmung der Krümmung des äußeren Umfangs der tierischen Herzklappe entspricht.

9. Biologische Prothese (10) nach einem der Ansprüche 2 bis 8, **dadurch gekennzeichnet, dass** das Verbindungsstück (4), das geeignet ist, an einem zweiten Ende des intraparietalen Stiels (2) der intraparietalen Verstärkungsvorrichtung befestigt zu werden, durch einen gekrümmten Stab ausgeführt ist, wobei die Krümmung der Krümmung des äußeren Umfangs der tierischen Herzklappe entspricht.

10. Biologische Prothese (10) nach Anspruch 1, **dadurch gekennzeichnet, dass** der intraparietale Stiel (2) der intraparietalen Verstärkungsvorrichtung (1) im Inneren der röhrenförmigen äußeren Wand (12) der tierischen Herzklappe entlang der Schnittlinien dieser Wand (12) mit den Verbindungen (13) der tierischen Herzklappe angeordnet ist.

11. Biologische Prothese (10) nach einem der Ansprüche 1, 2, 7, 8 und 9, **dadurch gekennzeichnet, dass** der Fuß (3) und/oder das Verbindungsstück (4), das geeignet ist, an den Enden des intraparietalen Stiels (2) der intraparietalen Verstärkungsvorrichtung befestigt zu werden, von einem Teflongewebe bedeckt ist/sind.

12. Biologische Prothese (10) nach Anspruch 11, **dadurch gekennzeichnet, dass** die intraparietale Verstärkungsvorrichtung, insbesondere der intraparietale Stiel (2), sich vollständig in dem biologischen Gewebe der tierischen Herzklappe befindet und sich niemals in Kontakt mit dem einseitig gerichteten Blutstrom befindet, der durch die röhrenförmige äußere Wand der tierischen Herzklappe fließt.

13. Biologische Prothese (10) nach Anspruch 12, **dadurch gekennzeichnet, dass** die gesamte intraparietale Verstärkungsvorrichtung sich vollständig in dem biologischen Gewebe der tierischen Herzklappe befindet und sich niemals in Kontakt mit dem einseitig gerichteten Blutstrom befindet, der durch die röhrenförmige äußere Wand der tierischen Herzklappe fließt.
